# EUROPEAN PATENT SPECIFICATION

(11) **EP 1 049 470 B1**
(45) Date of publication and mention of the grant of the patent: **03.01.2007**
(21) Application number: 99902409.4
(22) Date of filing: 22.01.1999
(51) Int. Cl.: A61K 31/445, A61K 9/22, A61K 9/16, A61K 9/48

(54) **EXTENDED RELEASE TIAGABINE FORMULATIONS WITH REDUCED SIDE-EFFECTS**
TIAGABINE ENTHALTENDE MIT VERLÄNGERTER FREISETZUNG FORMULIERUNGEN MIT REDUZIERTEN NEBENEFFEKTEN
FORMULATIONS DE TIAGABINE A LIBERATION PROLONGEE ENTRAINANT MOINS D'EFFETS SECONDAIRES

(30) Priority: 22.01.1998 US 72130 P
(43) Date of publication of application: 08.11.2000
(73) Proprietor: ABBOTT LABORATORIES, Abbott Park, IL 60064-6050 (US)
(72) Inventor: ALVAREZ, Francisco, J., Wilmington, DE 19808 (US); SLADE, Russell, T., Lindenhurst, IL 60046 (US); APFELBAUM, Kathleen, M., Lake Villa, IL 60046 (US); BROWN, David, M., Lake Villa, IL 60046 (US); GUSTAVSON, Linda, E., Evanston, IL 60203 (US)
(74) Representative: Modiano, Micaela Nadia
(86) International application number: PCT/US1999/001242
(87) International publication number: WO 1999/037302

(56) References cited:
- WO-A-95/05808
- WO-A-95/29665
- WO-A-96/34606
- WO-A-99/06045

## Description

### Background of the Invention

Tiagabine is used for controlling seizures in certain types of epilepsy. However, tiagabine sometimes produces uncomfortable side-effects, which if severe, may lead to discontinuation of anti-epileptic therapy with the compound. Certain of the side-effects are related to the central nervous system that are associated with reduced tolerance for the drug. Examples of such side effects include, ataxia, dizziness, headache, pharyngitis, and abnormal vision and thought processes.

To minimize adverse events from tiagabine therapy, one initiates tiagabine therapy by administering small doses, and then slowly and carefully increasing (titrating) the dosage to the optimal therapeutic level. This delays the time required to reach the therapeutically optimal tiagabine plasma concentration. The delay is detrimental not only because of the delay in controlling the seizures, but because side-effects may develop before the optimum treatment level is reached.

Extended release formulations of medicaments are well-known. However, such compositions have generally been utilized to prevent of deactivation of the drug in the intestinal tract before absorption into the blood stream, to maintain a more constant concentration of the drug in the blood, or to allow drug administration at less frequent intervals.

WO 95/05808 discloses a tablet for the systemic delivery of tiagabine containing an oil which is liquid at room temperature for controlling the release of tiagabine. Optionally, other agents may be added such as sodium alginate, glyceryl behenate, polyethylene glycol or hydroxypropyl methylcellulose. WO 96/34606 discloses pharmaceutical compositions containing tiagabine, antioxidants and a pharmaceutically acceptable carrier such as hydroxypropyl methylcellulose. WO 99/06045, which is prior art under the terms of Article 54(3) and (4) EPC, discloses controlled release compositions containing tiagabine dispersed in a rate controlling polymeric matrix comprising at least one rate controlling polymer such as polyethylene oxide.

### Summary of the Invention

Applicants have found that administering an extended release formulation of tiagabine to a patient produces fewer side-effects for the patient. As a further advantage, the extended release formulation also requires little or no titration phase, thus minimizing the time required for achieving seizure control. Furthermore, the extended release formulation may allow for a reduced frequency of dosing, e.g., once a day dosing.

Extended release compositions of tiagabine may be prepared in several forms, including matrix tablets and microparticulated pellets. Matrix tablets as described below contain high molecular weight polyethylene oxide which is a hydrophilic polymer. Optional hydrophilic reagents may be added to modify the rate of release of the active ingredient.

Multiparticulate pellets of tiagabine may be prepared by encapsulating the drug with hydrophobic materials selected from waxes, glyceryl behenate, triglycerides or mixtures of these materials. Again, optional hydrophilic reagents may be added to modify the rate of release of the active ingredient. The encapsulation process comprises suspending the drug in the molten material and forming small spherical particles as the molten material comes into contact with a disk rotating at high speed. The formed particles are cooled to solidify the hydrophobic encapsulating particles.

### Brief Description of the Figures

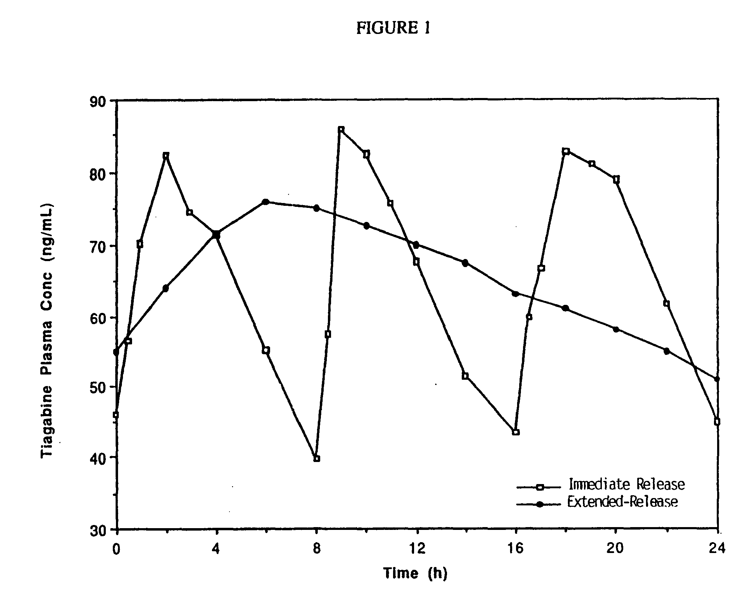

Figure 1 shows the desired release profile of the extended release tiagabine hydrochloride tablet (12 mg, QD) as compared to the immediate release profile (4 mg, TID).

### Detailed Description of the Invention

One embodiment of the invention is an extended-release composition as defined below comprising tiagabine combined in a matrix with high molecular weight polyethylene oxide (Polyox). One preferred formulation is a tablet form. In an optional embodiment, other hydrophilic reagents, such as hydroxypropylmethyl cellulose or hydroxypropyl cellulose, for example, may be employed to modify the rate of release of the active ingredient.

Another embodiment of the invention is an extended release formulation as defined below comprising tiagabine encapsulated in the formulation with a hydrophobic material selected from a wax, glyceryl behenate, triglycerides or mixtures of these materials. One preferred hydrophobic material is glyceryl behenate. A preferred formulation is a capsule form. In an optional embodiment, other hydrophilic reagents, such as hydroxypropyl methyl cellulose or hydroxypropyl cellulose, for example, may be employed to modify the rate of release of the active ingredient.

The extended release formulation according to the invention is selected from the group consisting of:
a) a formulation comprising tiagabine or a pharmaceutically acceptable salt thereof combined in a matrix having
   from 0.5 to 30 wt.% tiagabine or a pharmaceutically acceptable salt thereof ;
   from 15 to 80 wt.% of polyethylene oxide having a molecular weight in the range of 100000 to 8000000;
   from 5 to 80 wt.% of a filler selected from the group consisting of microcrystalline cellulose, starch and sugars;
   from 0.1 to 4 wt.% of silicon dioxide;
   from 0.1 to 1.5 wt.% of a preservative selected from the group consisting of tocopherol, BHA and BHT;
   from 0.1 to 6 wt.% of a first lubricant selected from the group consisting of wax, stearic acid, mineral oil, stearate and a hydrogenated vegetable oil; and
   from 0.05 to 1 wt.% of a second lubricant selected from the group consisting of magnesium stearate, calcium stearate and talc; and
b) a formulation in which tiagabine or a pharmaceutically acceptable salt thereof is encapsulated with a hydrophobic material selected from the group consisting of waxes, glyceryl behenate, triglycerides and mixtures thereof, wherein tiagabine or a pharmaceutically acceptable salt thereof is encapsulated by suspending tiagabine or a pharmaceutically acceptable salt thereof in the molten material and forming small spherical particles as the molten material comes into contact with a disk rotating at high speed, and then cooling the particles to solidify the molten material.

In a preferred embodiment, the extended release formulation comprises tiagabine in a capsule form in which the tiagabine is encapsulated in the formulation with glyceryl behenate by suspending the drug in the molten material and forming small spherical particles as the molten material comes into contact with a disk rotating at high speed, with subsequent cooling of the particles and subsequent formulation into capsules.

Preferably, the formulation in which tiagabine is encapsulated with a hydrophobic material comprises from 0.5 to 30 wt.% tiagabine or a pharmaceutically acceptable salt thereof encapsulated in a formulation having
from 50 to 99.5 wt.% of a hydrophobic ingredient selected from the group consisting of glyceryl behenate, bees wax, carnauba wax, triglycerides and hydrogenated vegetable oil;
from 0.1 to 4 wt.% of silicon dioxide;
from 0.1 to 1.5 wt.% of a preservative selected from the group consisting of tocopherol, BHA and BHT.

### Process to Prepare Tablets

In order to prepare solid, shaped dosage forms from fine particles or powders comprising therapeutic agents, it is generally necessary to process the powders in a manner that improves flowability, cohesiveness and other characteristics which will enable the resulting material to be fabricated by conventional processes such as encapsulation, molding, tableting, etc. into a satisfactory unit form that can suitably deliver an agent to the patient.

Various processes have been developed for modifying starting powders or other particulate materials. Typically the powders are gathered together with a binder material into larger permanent free-flowing agglomerates or granules referred to collectively as a "granulation." For example, solvent-assisted "wet" granulation processes are generally characterized in that the powders are combined with a binder material and moistened with water or an organic solvent under conditions to result in formation of a wet granulated mass from which the solvent must then be evaporated. Alternatively, known "dry granulation" processes can be used, depend on milling schemes, to produce a suitable granulation.

A "direct compression" process has, in limited cases, provided a simpler and more economical means of preparing compressed dosage forms. In such a process, the active ingredient is combined with a binder-diluent or vehicle which itself is characterized in having the requisite properties for tableting, such as flowability, appropriate particle size distribution, binding ability, acceptable bulk and tap density and dissolution properties, so that the resulting blend can be "directly" provided to a die cavity or mold for compaction, without prior granulation. See US 5,273,758 to Shangraw; "Compressed Tablets by Direct Compression," Pharmaceutical Dosage Forms. 2d Ed., v. 1, pp. 195-246 (1989).

A suitable direct compression vehicle for a given application is preferably also tailored, for example, to be compatible with the active ingredient; to resist physical or chemical change on aging; to be air, moisture and heat-stable; have sufficient capacity for the active ingredient in the dosage form; to accept colorants uniformly when necessary; and not to interfere with biological availability.

Materials employed by the art which to varying degrees fulfill the requirements of a direct compression vehicle include water soluble materials such as various forms of lactose (e.g., spray-dried lactose, Fast Flow" lactose, anhydrous lactose), as well as sucrose, dextrose, sorbitol, mannitol and maltodextrin, and relatively insoluble materials such as microcrystalline cellulose (e.g., Avicel"), starch, dicalcium phosphate dihydrate, and calcium carbonate.

However, such materials, while often comprising a relatively large proportion by weight of the tableted formulation in order to impart full advantage of their compression properties, nevertheless in themselves are generally insufficient to regulate the rate of disintegration of the dosage form or release of the medicament, and therefore must often be accompanied by various additional excipients having such a rate-control effect, the latter which (given practical limitations on the size of the dosage form) may be confined to low concentrations at which the rate control effect is not completely satisfactory.

Polyox is a nonionic homopolymer of the formula -(-O-CH2-CH2-)ₙ- , wherein n represents the average number of oxyethylene groups, n generally being from about 2,000 to about 100,000. It is a water soluble resin which is available as a white powder in several grades which vary in viscosity profile when dissolved in water. National Formulary XVII, pp. 1963-1964 (1990). Molecular weights range from about 100,000 to about 8,000,000, corresponding to a viscosity range of under about 200 cps for a 5% aqueous solution of the lower molecular weight polymers to about 7,000 to 10,000 cps for a 1% solution of the higher molecular weight polymers. Polyethylene oxide resins are commercially available under the tradename Polyox® from Union Carbide Corporation. Polyox® WSR 303 has an average molecular weight of about 7,000,000, and a 1% aqueous solution thereof at 25 °C has a viscosity of about 7,200 to 10,000 cps on a Brookfield RVF, No. 2 spindle at 2 rpm, and a pH of 8 to 10.

The use of a particular molecular weight polyethylene oxide polymer as a binder material will depend on the desired disintegration or release rate characteristics to be imparted to the prepared dosage form. In general, lower molecular weight polyethylene oxide polymers, i.e. having MW of up to about 300,000, e.g., Polyox® N80. may be selected to prepare tablets from which the medicament is released within a relatively short time period. Sustained release dosage forms may be prepared from the higher molecular weight polymers, i.e. having MW higher than about 300,000, especially about 2,000,000 to 7,000,000 (e.g., Polyox® 303 and Polyox® WSR Coagulant). It is contemplated that mixtures of varying molecular weight polymers may also be employed as a matrix system to obtain the desired tablet release properties, and such mixtures may comprise respective amounts of the various polyethylene oxide polymers as shall be within the skill of the worker in the art to ascertain to provide the appropriate release pattern.

Other optional components of the matrix compositions of the invention include various fillers, binders, disintegrants, diluents, hydrophilic polymers, etc., including cellulose ethers, such as HPMC, and waxy substances, as well as minor amounts of various lubricants such as talc, colloidal silicon dioxide, stearic acid or metal stearates, etc., and colorants, sweeteners, antioxidants, and the like.

Suitable fillers are microcrystalline cellulose, starch and sugars such as lactose and mannitol, which are employed in amounts from 5% to 80% of the blend. Higher molecular weight polyethylene oxide polymers, for example, Polyox® 303 and Polyox® WSR Coagulant, are employed in amounts from 15% to 80% of the blend. Silicon dioxide is employed in amounts from 0.1 % to 4% of the blend. Other lubricants, selected from waxes, hydrogenated vegetable oil, stearic acid, calcium stearate, magnesium stearate, mineral oil, and talc, are employed in the percentages of the blend disclosed above. The antioxidants Vitamin E, BHA, and BHT are employed in amounts from 0.1 % to 1.5% of the blend.

The dosage forms may be prepared by a direct compression process; that is, the process consists essentially of, the steps of (i) dry blending particles comprising 15 to 80 wt.%, and preferably 20 to 60 wt.%, of polyethylene oxide with 0.5 to 30% of tiagabine, the therapeutic medicament, as well as other optional excipients, and (ii) providing the resulting mixture to a compression machine, and applying sufficient pressure to the composition to form a unitary dosage form.

The medicament may be employed in powder, crystalline, or other form, and typically need not be compounded to an amorphous or other type granulated form.

In one embodiment, the polyethylene oxide and medicament and other optional ingredients are dry blended, i.e. in the absence of added solvents or heat, to produce a free-flowing material wherein the medicament is well dispersed in the polyethylene binder-matrix. The mixture is then provided to, for example, a tableting machine and a compression force of about 0.5 to 10 tons is applied to prepare a tablet dosage form. In such a tablet, the medicament is generally evenly dispersed throughout the polyethylene oxide binder, and which is free of solvent residues.

As used herein, the term "tablet" refers to a compressed body which is composed of a plurality of discrete particles, and includes pills, lozenges, dragee cores, capsule slugs, molded forms, and the like.

In another embodiment, a hydrophobic ingredient as defined above, such as glyceryl behenate, beeswax, carnauba wax, triglycerides, and hydrogenated vegetable oils, and medicament and other optional ingredients are blended in a heated mixer. The molten material is then dropped at a rate of about 30 mL/min to about 300 mL/min, and preferably about 100 mL/min, onto a disk rotating from about 1000 to 5000 rpm, and preferably about 3000 rpm. The droplets thrown off the disk are solidified by air-cooling and collected. In a preferred embodiment, glyceryl behenate is the hydrophobic ingredient. These particles are then placed into a gelatin capsule to form a capsule dosage form.

As used herein, the term "capsule" refers to a gelatin capsule which is filled with a plurality of discrete particles formed from a molten blend of tiagabine plus a hydrophobic ingredient and other optional ingredients.

The expression "therapeutic medicament" or "drug" shall include any physiologically or pharmacologically active substance that produces a local or systemic effect(s) in animals, which include warm-blooded mammals, humans, primates, etc.

The term "physiological" as used herein denotes the administration of a drug to effect normal levels and functions. The term "pharmacological" denotes variations in response to the amount of drug administered to the host. The devices have found a particular use as vehicles for various human and animal drugs, particularly for the oral administration thereof, although for other systems as well, including systems such as buccal, implant, nose, artificial gland, rectum, cervical, intrauterine, occular, arterial, venous, ear and the like, may be manufactured according to the process of the invention.

Compositions according to the invention may comprise tiagabine in free base form, or in a pharmaceutically acceptable salt form, preferably HCl. The chemical name for tiagabine is (-)-(R)-1-[4,4-bis(3-methyl-2-thienyl)-3-butenyl]nipecotic acid. Unless otherwise expressly indicated the chemical substances used are in the National Formulary or the U.S. Pharmacopeia.

### Examples of formulations

### Example 1: Matrix Formulations Comprising (Polyox)

Ingredients shown in the table below were mixed in a V-blender for about 5-30 minutes. The powder was then compressed into 300 mg tablets by means of a tablet press, applying about 500 to 3500 pounds of compression force.

Four different blends were prepared, as shown, each having a different amount of tiagabine per table. The A4, A12, A20 and A32 tablets contained 4,12,20 and 32 mg of tiagabine per tablet, respectively.

| Item | Name | % of Formula | | | |
|---|---|---|---|---|---|
| | | A4 | A12 | A20 | A32 |
| 1 | Microcrystalline cellulose (Avicel PH 102) | 32.1 | 28.7 | 25.3 | 20.2 |
| 2 | Colloidal Silicon Dioxide, NF (Cab-O-Sil M5) | 0.8 | 1.4 | 2.0 | 2.9 |
| 3 | Vitamin E (dl-alpha tocopherol), USP | 0.5 | 0.5 | 0.5 | 0.5 |
| 4 | Water, Purified, USP (distilled) | qs | qs | qs | qs |
| 5 | Tiagabine Hydrochloride | 1.4 | 4.2 | 7.0 | 11.2 |
| 6 | Polyox WSR Coagulant (MW 5MM) | 60.0 | 60.0 | 60.0 | 60.0 |
| 7 | Wax, Hydrogenated Vegetable Oil (Sterotex K) | 5.0 | 5.0 | 5.0 | 5.0 |
| 8 | Magnesium Stearate, NF, Impalpable powder | 0.2 | 0.2 | 0.2 | 0.2 |

### Example 2: Additional Matrix Formulations Comprising Polyox

Ingredients shown in the table below were mixed in a V-blender for 5-30 minutes. The powder was then compressed into 300 mg tablets by means of a tablet press, applying about 500 to 3500 pounds of compression force.

Three different blends were prepared, as shown, each having a different amount of tiagabine per blend. Tablets were prepared from each blend, and each table contained 10 mg of tiagabine per tablet.

The tablets from this example were used in the in vivo study described in Example 5 below.

| Item | Name | % per Tablet | | |
|---|---|---|---|---|
| | | lot-172 | lot-173 | lot-174 |
| 1 | Cellulose (Avicel PH 102) | 70.7 | 25.7 | 30.3 |
| 2 | Tiagabine HCl Tablet Pre-Mix* | 9.4 | 9.4 | 4.7 |
| 3 | Polyethylene Oxide (Polyox WSR 303) | 15.0 | 60.0 | 60.0 |
| 4 | Wax, Hydrogenated Veg. Oil (Sterotex K) | 5.0 | 5.0 | 5.0 |
| | | 100.0 | 100.0 | 100.0 |

| | | | | |
|---|---|---|---|---|
| *Premix contained 82% tiagabine HCl and 18% SiO₂ | | | | |

### Example 3: Multiparticulate Formulations Comprising Hydrophobic Excipients

Ingredients shown in the table below were blended in a heated mixer, and the molten material was dropped and the rate of 100 mL per minute onto a disk rotating at 3000 rpm. The droplets thrown off the disk were air cooled, and the solidified particles were collected.

Two different blends were prepared, as shown, each having a different amount of tiagabine per unit weight of particulate material. The F3 and F4 tablets contained 5 and 21 mg of tiagabine, respectively, per 100 mg of particulate material.

| Item | Name | % per Tablet | |
|---|---|---|---|
| | | F-3 | F-4 |
| 1 | Glyceryl Behenate, Compritol 888 ATO | 93.1 | 74.0 |
| 2 | d-alpha tocopherol (Vitamin E) | 0.5 | 0.5 |
| 3 | Tiagabine HCl Tablet Pre-Mix | 6.4 | 25.5 |

### Example 4: Dissolution Profiles of Various Tiagabine Formulations

Formulations of reference immediate release formulations and the experimental formulations described prepared as described in Examples 1-2 above were tested in a multiple position dissolution stirrer such as that described at USP p.1244, which was equipped with a Teflon paddle (50 rpm) in each of six vessels. A dissolution medium comprising 900 mL. of deaerated and distilled water was maintained at 37°C ± 0.5 °C. A tablet was sequentially dropped into each vessel. Stirring and timing (time zero) was commenced as the first tablet hit the bottom of the vessel (under the paddle).

At regular intervals, aliquots of test solution were withdrawn from each of the vessels in the order in which the tablets were originally dropped, using a stainless steel cannula. The aliquots were withdrawn from a point midway between the surface of the dissolution medium and the top of the paddle and not less than 1 cm. from each vessel wall. The amount of tiagabine present in each of the vessels was calculated by reference to standard solutions using HPLC. Dissolution profiles of the formulations from Examples 1-2 above are shown in Graphs 1-2 below.

Graph 1 shows the cumulative amount of tiagabine dispensed over a prolonged period of time from an extended release matrix formulation using high molecular weight polyethylene oxide.

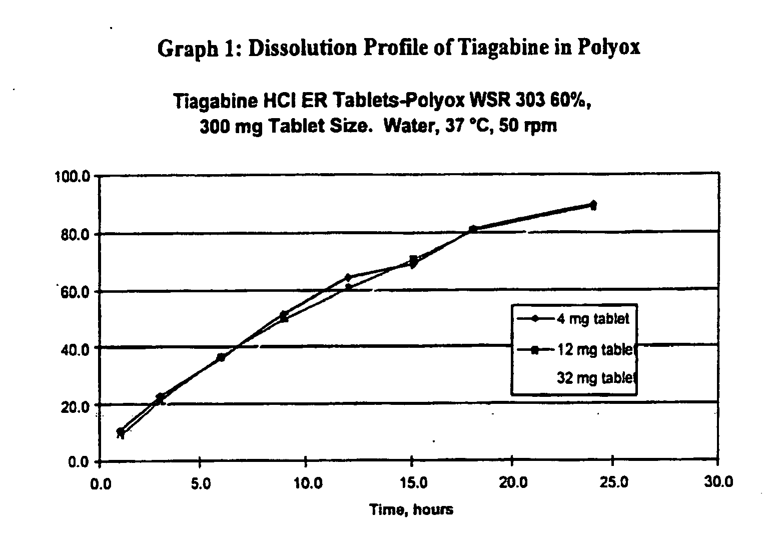

Graph 2 shows the cumulative amount of tiagabine dispensed over a prolonged period of time from an extended release formulation in a multiparticulate system.

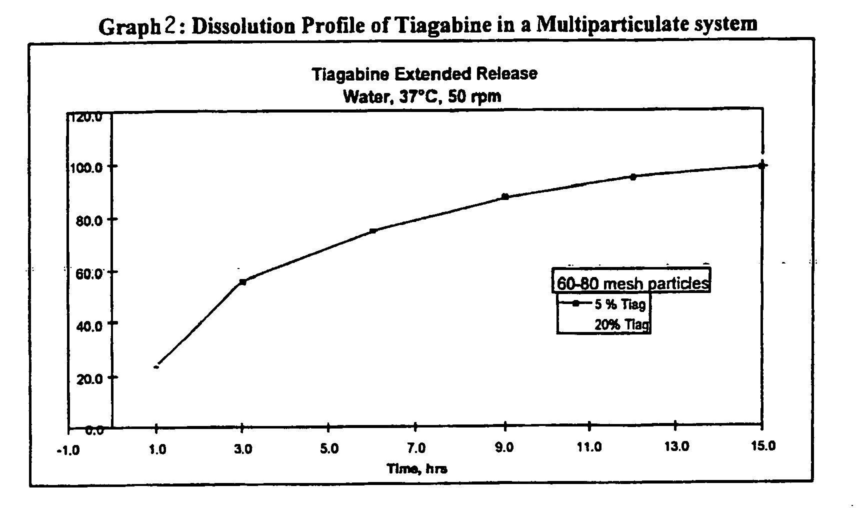

As Graphs 1-2 demonstrate, no more than 80% of the tiagabine was released in less than 5 hours. By contrast, a standard immediate release formulation of tiagabine (Gabitril^{™}, obtained from Novo-Nordisk) was found to release greater than 80% of the drug within 60 minutes or less.

### Example 5: In Vivo Comparison Between Extended Release Tiagabine and Immediate Release Formulation

Several formulations containing high molecular weight polyethylene oxide were compared in a human bioavailability study where it was found that they were bioequivalent to the immediate release dosage form. One Tiagabine extended release tablet containing 10 mg was administered orally to 16 fasting healthy male subjects. Of these, 13 completed the study. A summary of the pharmacokinetic results (mean ± SD) is presented in the Table below. The pharmacokinetic profiles showed a significant decrease in Cₘₐₓ and an increase in tₘₐₓ for the extended release formulation.

**Pharmacokinetic Profiles of Extended Release Formulations Compared with Gabitril^{™} Control**

| Regimen | Cₘₐₓ (mg/mL) | Tₘₐₓ (hr) | AUC₀₋ (mg hr/mL | t_{1/2} (hr)† |
|---|---|---|---|---|
| Lot 172 | 70.8 ± 10.8* | 3.7 ± 0.6* | 1283 ± 279 | 9.1 |
| Lot 173 | 60.7 ± 10.6* | 6.8 ± 5.3* | 1266 ± 268** | 9.7 |
| Lot 174 | 54.8 ± 8.8* | 8.1 ± 3.5* | 1320 ± 272 | 11.2 |
| Gabitril control | 241.3 ± 59.1 | 0.7 ± 0.2 | 1387 ± 277 | 9.5 |

| | | | | |
|---|---|---|---|---|
| † Harmonic mean. * Statistically significant difference as compared to control (p 0.05). ** Statistically significant difference as compared to control (p 0.05) for analysis of log-transformed AUC₀₋ only. | | | | |

However, and unexpectedly, the number of adverse events observed during the clinical study, particularly those related to the central nervous system, were fewer than those observe with the immediate release formulation (see Table below). The side effects profile observed with the immediate release tiagabine formulation are consistent with those observed with these type of formulations in other clinical studies. The data indicate that an extended release formulation can provide comparable therapy with fewer adverse events.

**Adverse Events Occurring in Two or More Subjects for Any One Regimen of Tiagabine**

| Extended Release Formulation: Tiagabine Tablet, | | | | |
|---|---|---|---|---|
| Adverse Event | lot-172 N=15 | 10 mg lot-173 N=15 | lot-174 N=14 | Reference N=15 |
| Ataxia | 0 | 0 | 0 | 2(13.3%) |
| Dizziness | 2(13.3%) | 0 | 0 | 11(73.3%) |
| Headache | 2(13.3%) | 3(20.0%) | 2(14.3%) | 1(6.7%) |
| Thinking Abnormal | 0 | 0 | 0 | 5(33.3%) |
| Pharyngitis¹ | 2(13.3%) | 1(6.7%) | 0 | 1(6.7%) |
| Abnormal Vision | 0 | 0 | 0 | 2(13.3%) |
| ¹ In each case, the event was considered to have no relationship to tiagabine | | | | |

### Example 6: Additional In Vivo Comparison Between Extended Release Tiagabine and Immediate Release Formulation

A 12 milligram tiagabine hydrochloride extended release tablet, Formulation A12 in Example 1, was compared to a 4 milligram immediate release tablet. The extended release tablet was administered each morning for five (5) days under fasting conditions while the immediate release tablet was administered every eight (8) hours for five (5) consecutive days under nonfasting conditions.

A total of fourteen healthy subjects were tested in two stages. In the first stage, seven subjects received the extended release tablet and seven subjects received the immediate release tablet for the five days. A washout interval of seven days separated the last dose of stage 1 from the first dose of stage 2. In stage 2, the seven subjects that received the extended release tablet in 1 now received the immediate release tablet and the seven subjects who initially received the immediate release tablet now received the extended release tablet.

Blood samples were collected for determination of plasma tiagabine concentrations at 0 hours, 0.5, 1, 2, 3, 4, 6, 8, 8.5, 9, 10, 11, 12, 14, 16, 16.5, 17, 18, 19, 20, 22, and 24 hours after the morning dose on Day5 in each period. Additional blood samples were collected before the morning dose on Days 1, 3, and 4 in each period.

Blood samples were placed in an ice bath and protected from light. Plasma was separated from whole blood within one hour of collection by refrigerated centrifugation and stored frozen (≤20°C).

Plasma concentrations of tiagabine were determined using a validated liquid chromatography method with tandem mass spectrometric detection (LC/MS/MS). A monomethyl analog of tiagabine was used as an internal standard.

A summary of the pharmacokinetic results (mean ± SD) is presented in the Table below. The pharmacokinetic profiles showed a significant decrease in Cₘₐₓ and an increase in tₘₐₓ for the extended release tablet.

**Pharmacokinetic Profiles of Extended Release Formulations Compared with Gabitril^{™} Control**

| Regimen | Cₘₐₓ (ng/mL) | Tₘₐₓ (hr) | AUC₀₋₂₄ (ng hr/mL | Cmin (ng/mL) |
|---|---|---|---|---|
| Extended Release | 84.0 ± 26.1* | 4.7 ± 5.2* | 1354 ± 551 | 34.5 ± 21.3 |
| Immediate Release | 105.3 ± 17.0 | 1.6 ± 1.1* | 1573 ± 311 | 37.4 ± 11.0 |

| | | | | |
|---|---|---|---|---|
| * Statistically significant difference as compared to immediate release tablet (p=0.0069). | | | | |

The present invention relates to extended release formulations of tiagabine and its salts. In particular, extended release formulation of tiagabine hydrochloride are provided for oral administration to mammals, and in particular, human patients. Preferred tiagabine formulations provide an extended-release oral administration to human patients, comprising from about 4 to about 80 mg tiagabine or a salt thereof, said formulation providing a mean maximum plasma concentration of tiagabine from about 10 to 1000 ng/mL from a mean of about 2 to 8 hours after administration, and a mean minimum plasma concentration of tiagabine from about 1 to 700 ng/mL from a mean of about 22 to 26 hours after repeated administration every 24 hours through steady-state conditions.

An extended release formulation showing the release profile above comprising tiagabine may be combined in a matrix with a hydrophilic polymer selected from the group consisting of high molecular weight polyethylene oxides as described above.

An extended release formulation comprising tiagabine may be encapsulated in a formulation with a hydrophobic material selected from the group consisting of waxes, glyceryl behenate, triglycerides and mixtures thereof, as described above.

A preferred embodiment of the present invention includes an extended-release oral tiagabine tablet, comprising from about 4 to about 80 mg tiagabine or a salt thereof, said tablet providing a mean maximum plasma concentration of tiagabine from about 10 to 1000 ng/mL from a mean of about 2 to 8 hours after administration, and a mean minimum plasma concentration of tiagabine from about 1 to 700 ng/mL from a mean of about 22 to 26 hours after repeated administration every 24 hours through steady-state conditions.

An extended release tablet comprising tiagabine may be combined in a matrix with a hydrophilic polymer selected from the group consisting of high molecular weight polyethylene oxide as described above.

An extended release tablet comprising tiagabine may be encapsulated in a formulation with a hydrophobic material selected from the group consisting waxes, glyceryl behenate, triglycerides and mixtures thereof as described above.

## Claims

1. An extended release formulation selected from the group consisting of:
a) a formulation comprising tiagabine or a pharmaceutically acceptable salt thereof combined in a matrix having
from 0.5 to 30 wt.% tiagabine or a pharmaceutically acceptable salt thereof ;
from 15 to 80 wt.% of polyethylene oxide having a molecular weight in the range of 100000 to 8000000;
from 5 to 80 wt.% of a filler selected from the group consisting of microcrystalline cellulose, starch and sugars;
from 0.1 to 4 wt.% of silicon dioxide;
from 0.1 to 1.5 wt.% of a preservative selected from the group consisting of tocopherol, BHA and BHT;
from 0.1 to 6 wt.% of a first lubricant selected from the group consisting of wax, stearic acid, mineral oil, stearate and a hydrogenated vegetable oil; and
from 0.05 to 1 wt.% of a second lubricant selected from the group consisting of magnesium stearate, calcium stearate and talc; and
b) a formulation in which tiagabine or a pharmaceutically acceptable salt thereof is encapsulated with a hydrophobic material selected from the group consisting of waxes, glyceryl behenate, triglycerides and mixtures thereof, wherein tiagabine or a pharmaceutically acceptable salt thereof is encapsulated by suspending tiagabine or a pharmaceutically acceptable salt thereof in the molten material and forming small spherical particles as the molten material comes into contact with a disk rotating at high speed, and then cooling the particles to solidify the molten material.

2. The formulation according to claim 1 wherein the excipient is glyceryl behenate in a capsule form in which the tiagabine or a pharmaceutically acceptable salt thereof is encapsulated in the formulation with glyceryl behenate by suspending the drug in the molten material and forming small spherical particles as the molten material comes into contact with a disk rotating at high speed, with subsequent cooling of the particles and subsequent formulation into capsules.

3. The formulation according to claim 1 comprising from 0.5 to 30 wt.% tiagabine or a pharmaceutically acceptable salt thereof encapsulated in a formulation having
from 50 to 99.5 wt.% of a hydrophobic ingredient selected from the group consisting of glyceryl behenate, bees wax, carnauba wax, triglycerides and hydrogenated vegetable oil;
from 0.1 to 4 wt.% of silicon dioxide;
from 0.1 to 1.5 wt.% of a preservative selected from the group consisting of tocopherol, BHA and BHT.

4. The formulation of claim 1, wherein said hydrophobic material is glyceryl behenate.

5. The formulation according to claim 1 or 4 wherein the formulation b) is in the form of a capsule containing tiagabine or a pharmaceutically acceptable salt thereof encapsulated with said hydrophobic material.

6. The formulation according to claim 1 wherein the formulation a) is shaped in a tablet form.

7. Use of a formulation according to claim 1 for the manufacture of an extended release medicament.

## Patentansprüche

1. Eine Formulierung mit verlängerter Freisetzung gewählt aus der Gruppe bestehend aus:
a) einer Formulierung umfassend Tiagabin oder ein pharmazeutisch verträgliches Salz davon, kombiniert in einer Matrix, die folgendes hat:
von 0,5 bis 30 Gewichtsprozent Tiagabin oder ein pharmazeutisch verträgliches Salz davon;
von 15 bis 80 Gewichtsprozent Polyethylenoxid mit einem Molekulargewicht in dem Bereich von 100000 bis 8000000;
von 5 bis 80 Gewichtsprozent eines Füllstoffs gewählt aus der Gruppe bestehend aus mikrokristalliner Zellulose, Stärke und Zuckern;
von 0,1 bis 4 Gewichtsprozent Siliziumdioxid;
von 0,1 bis 1,5 Gewichtsprozent eines Konservierungsstoffs gewählt aus der Gruppe bestehend aus Tocopherol, BHA und BHT;
von 0,1 bis 6 Gewichtsprozent eines ersten Schmiermittels gewählt aus der Gruppe bestehend aus Wachs, Stearinsäure, Mineralöl, Stearat und einem hydrierten pflanzlichen Öl; und
von 0,05 bis 1 Gewichtsprozent eines zweiten Schmiermittels gewählt aus der Gruppe bestehend aus Magnesiumstearat, Kalziumstearat und Talkum; und
b) einer Formulierung, in welcher Tiagabin oder ein pharmazeutisch verträgliches Salz davon mit einem hydrophoben Material eingekapselt ist, gewählt aus der Gruppe bestehend aus Wachsen, Glycerylbehenat, Triglyceriden und Mischungen daraus, worin Tiagabin oder ein pharmazeutisch verträgliches Salz davon eingekapselt wird durch Suspendieren von Tiagabin oder eines pharmazeutisch verträglichen Salzes davon in dem geschmolzenen Material, und Bilden von kleinen kugelförmigen Partikeln, wenn das geschmolzene Material mit einer Scheibe, die sich bei hoher Geschwindigkeit dreht, in Kontakt kommt, und dann Abkühlen der Partikel, um das geschmolzene Material zu verfestigen.

2. Die Formulierung gemäß Anspruch 1, worin das Bindemittel Glycerylbehenat in einer Kapselform ist, in welcher das Tiagabin oder ein pharmazeutisch verträgliches Salz davon in der Formulierung mit Glycerylbehenat eingekapselt wird durch Suspendieren des Arzneistoffs in dem geschmolzenen Material und Bilden von kleinen kugelförmigen Partikeln, wenn das geschmolzene Material mit einer Scheibe, die sich bei hoher Geschwindigkeit dreht, in Kontakt kommt, mit nachfolgendem Abkühlen der Partikel und nachfolgender Formulierung in Kapseln.

3. Die Formulierung gemäß Anspruch 1, die von 0,5 bis 30 Gewichtsprozent Tiagabin oder ein pharmazeutisch verträgliches Salz davon umfaßt, eingekapselt in einer Formulierung, die folgendes hat:
von 50 bis 99,5 Gewichtsprozent eines hydrophoben Inhaltsstoffs gewählt aus der Gruppe bestehend aus Glycerylbehenat, Bienenwachs, Carnaubawachs, Triglyceriden und hydriertem pflanzlichen Öl;
von 0,1 bis 4 Gewichtsprozent Siliziumdioxid;
von 0,1 bis 1,5 Gewichtsprozent eines Konservierungsstoffs gewählt aus der Gruppe bestehend aus Tocopherol, BHA und BHT.

4. Die Formulierung von Anspruch 1, worin das hydrophobe Material Glycerylbehenat ist.

5. Die Formulierung gemäß Anspruch 1 oder 4, worin die Formulierung b) in der Form einer Kapsel ist, die Tiagabin oder ein pharmazeutisch verträgliches Salz davon enthält, eingekapselt mit dem hydrophoben Material.

6. Die Formulierung gemäß Anspruch 1, worin die Formulierung a) in einer Tablettenform ausgestaltet ist.

7. Verwendung einer Formulierung gemäß Anspruch 1, für die Herstellung eines Medikaments mit verlängerter Freisetzung.

## Revendications

1. Formulation à libération modifiée choisie dans le groupe constitué par :
a) une formulation comprenant de la tiagabine ou un sel pharmaceutiquement acceptable de celle-ci combiné dans une matrice ayant :
de 0,5 à 30 % en poids de tiagabine ou d'un sel pharmaceutiquement acceptable de celle-ci ;
de 15 à 80 % en poids d'oxyde de poly(éthylène) ayant une masse moléculaire dans la gamme de 100 000 à 8 000 000 ;
de 5 à 80 % en poids d'une charge choisie dans le groupe constitué de cellulose microcristalline, d'amidon et de sucres ;
de 0,1 à 4 % en poids de dioxyde de silicium ;
de 0,1 à 1,5 % en poids d'un conservateur choisi dans le groupe constitué du tocophérol, BHA et BHT ;
de 0,1 à 6 % en poids d'un premier lubrifiant choisi dans le groupe constitué de cire, d'acide stéarique, d'huile minérale, de stéarate et d'une huile végétale hydrogénée ; et
de 0,05 à 1 % en poids d'un second lubrifiant choisi dans le groupe constitué d'un stéarate de magnésium, d'un stéarate de calcium et de talc ; et
b) une formulation dans laquelle de la tiagabine ou un sel pharmaceutiquement acceptable de celle-ci est encapsulé avec un matériau hydrophobe choisi dans le groupe constitué de cires, du béhénate de glycérile, de triglycérides et des mélanges de ceux-ci ; dans laquelle de la tiagabine ou un sel pharmaceutiquement acceptable de celle-ci est encapsulé en réalisant les étapes consistant à mettre en suspension de la tiagabine ou un sel pharmaceutiquement acceptable de celle-ci dans le matériau fondu et à former de petites particules sphériques lorsque le matériau fondu entre en contact avec un disque tournant à une vitesse élevée, puis à refroidir les particules pour solidifier le matériau fondu.

2. Formulation selon la revendication 1, dans laquelle l'excipient est du béhénate de glycérile sous forme de capsule dans laquelle la tiagabine ou un sel pharmaceutiquement acceptable de celle-ci est encapsulé dans la formulation avec du béhénate de glycérile en réalisant les étapes consistant à mettre en suspension le médicament dans le matériau fondu et à former de petites particules sphériques lorsque le matériau fondu entre en contact avec un disque tournant à une vitesse élevée, et à refroidir par la suite les particules et à les formuler ensuite en des capsules.

3. Formulation selon la revendication 1, comprenant de 0,5 à 30 % en poids de tiagabine ou d'un sel pharmaceutiquement acceptable de celle-ci encapsulé dans une formulation ayant
de 50 à 99,5 % en poids d'un ingrédient hydrophobe choisi dans le groupe constitué du béhénate de glycérile, de cire d'abeille, de cire de carnauba, de triglycérides et d'une huile végétale hydrogénée ;
de 0,1 à 4 % en poids de dioxyde de silicium ;
de 0,1 à 1,5 % en poids d'un conservateur choisi dans le groupe constitué du tocophérol, BHA et BHT.

4. Formulation selon la revendication 1, dans laquelle ledit matériau hydrophobe est du béhénate de glycérile.

5. Formulation selon la revendication 1 ou 4, dans laquelle la formulation (b) est sous la forme d'une capsule contenant de la tiagabine ou un sel pharmaceutiquement acceptable de celle-ci encapsulé avec ledit matériau hydrophobe.

6. Formulation selon la revendication 1, dans laquelle la formulation (a) est formée sous forme de comprimé.

7. Utilisation d'une formulation selon la revendication 1 pour la fabrication d'un médicament à libération modifiée.
